# EUROPEAN PATENT SPECIFICATION

(11) **EP 0 597 415 B1**
(45) Date of publication and mention of the grant of the patent: **17.03.1999**
(21) Application number: 93118055.8
(22) Date of filing: 08.11.1993
(51) Int. Cl.: A61K 33/26, A61K 31/70

(54) **Iron therapy using Saccharose octasulfate ferric complex**
Eisentherapie mit Saccharoseoctasulfateisenkomplex
complexe ferrique d'octasulfate de saccharose pour le traitement du syndrome de carence de fer

(30) Priority: 11.11.1992 IT MI922578
(43) Date of publication of application: 18.05.1994
(73) Proprietor: FLARER S.A., 6900 Lugano (CH)
(72) Inventor: Di Schiena, Michele Giuseppe, I-20080 Cisliano (Milano) (IT)
(74) Representative: Minoja, Fabrizio, Dr.

(56) References cited:
- EP-A- 0 299 910
- DE-A- 2 924 566
- DE-A- 3 844 065

## Description

The present invention relates to the use of saccharose octasulfate ferric complex in the therapy of sideropenic hypochromic anemias and of those conditions benefitting from an exogenous iron supply, such as pregnancy, breastfeeding, conditions of weariness, and some debilitating diseases.

EP-A- 0 299 910, in the name of Schering, discloses the compound saccharose octasulfate ferric complex, a process for the preparation of said compound and the use therof in diagnostics.

DE-2924566 discloses iron (III) complexes with **chondroitinsulfate** and the process for their preparation.

DE-3844065 discloses compositions of glycosidic iron (II) and iron (III) and the preparation thereof.

One of the main problems with the oral administration of iron-containing compounds resides in the poor gastric tolerability, whose manifestations are abdominal pains, nausea, and diarrhoea, and which affects the therapeutical efficacy due to the low compliance often resulting.

Iron complexes with proteins and polysaccharides have been proposed in order to reduce iron induced gastric lesions, however said complexes are difficult to preserve both as compounds and as pharmaceutical formulations, moreover they do not overcome satisfactorily the problem of gastric lesions.

Now it has been found, and it is the object of the present invention, that saccharose octasulfate ferric complex has peculiar characteristics making it preferable to the other iron compounds which are already known in therapy, particularly to those for the oral administration.

In the ferric compound according to the present invention, the metal is linked to an organic matrix, i.e. saccharose polysulfate, which proved capable of protecting the gastroenteral mucosa from a variety of damaging factors; said matrix, linked as an aluminium complex salt, has been used for some time in gastroenterology under the name sucralfate.

Saccharose octasulfate ferric complex is an odourless, tasteless, reddish-brown powder, which is virtually insoluble in water and in the common organic solvents; slowly soluble in diluted acids, such as diluted hydrochloric acid, a characteristic which makes the compound soluble in gastric juice, after the oral administration.

The iron content ranges from 37 to 43%; the octasulfate ion content varies from 18 to 24%, whereas the remainder consists of saccharose of a lower sulfation degree and mainly by hydroxy groups which are either directly linked to the iron or in the form of water linked to the Fe3⁺ atom.

The analytical characteristics of the compound confirm that the trivalent iron has only one valence linked to a sulfuric group, whereas the other two valences are engaged with the hydroxy groups as such or as water or, most likely, as a mixture thereof.

Another specific characteristic making saccharose octasulfate ferric complex preferable in iron therapy is the high iron content, allowing the preparation of tablets, pills, capsules of small size and therefore easy to swallow. This is, of course, a further advantage in the case of some patients.

On the contrary, the best known compounds used in therapy nowadays have an iron content generally ranging from 7 to 15%, thus involving an increase in the size of solid oral formulations. In fact, it should be noted that the therapeutical amount of a single iron administration is about 40 mg, independently of the pharmaceutical form.

The pharmaceutical forms suited for the administration of saccharose octasulfate acid ferric complex for the purposes of the present invention are those known in pharmaceutical technique, for example pills, tablets, sugar-coated pills, capsules, suspensions both oral and injectable, including those in which the compound is micronized or microdispersed in a suitable liquid or solid.

The therapeutical dose is generally 120-200 mg of ferric ion for one or more daily administration, preferably 20-40 mg of ferric ion for one or more daily administrations.

The following example illustrates a pharmaceutical composition according to the invention.

### EXAMPLE 1

### Oral capsules

Each capsule contains:

| | |
|---|---|
| Ferric saccharose octasulfate equivalent to 40 mg of trivalent iron | 100 mg |
| Lactose | 10 mg |
| Magnesium stearate | 10 mg |

the ingredients are mixed thoroughly and dispensed in gelatin capsules.

### EXAMPLE 2

### Ulcerogenic effect in rat

Ulcerogenic effect in rat was determined by comparing ferric saccharose octasulfate (hereinafter named Sucrafer) with Indomethacine (non steroidal antiinflammatory drug).

120-140 g Crl:CD(SD)BR male rats, starving from 9 hours, were orally treated with Sucrafer for two times at an interval of about 7 hours.

18 hours after the last treatment, the animals were sacrificed and the gastric lesions, if any, were examined.

The lesions were ranked according to the following conventional scheme:
- - spot ulcers:: 0 = no lesions
0.5 = 1-2 slight lesions
1 = 3-5 slight lesions
2 = > 5 slight lesions
2 = 1-2 deep lesions
3 = 3-5 deep lesions
4 = > 5 deep lesions
- - linear ulcers:: 0 = no lesions
1 = 1-2 slight lesions
2 = 3-5 slight lesions
3 = > 5 slight lesions
2 = 1-2 deep lesions
3 = 3-5 deep lesions
4 = > 5 deep lesions

Sucrafer was administered at the standard dose of 50 mg/kg per os (as Fe) and Indomethacine, used as standard, at the doses of 5 and 10 mg/kg per os.

The following table shows the absolute absence of ulcers in the rats treated with Sucrafer: the treatment with 5 and 10 mg/kg per os of Indomethacine caused the onset of ulcers in 80% and 100% of the animals, respectively.

**TABLE I**

| Treatment | Dose (mg/kg) | No. of rats | Total score | % of rats with ulcers |
|---|---|---|---|---|
| Controls | 0 | 10 | 0 | 0 |
| Indomethacine | 5 | 5 | 12 | 80 |
| Indomethacine | 10 | 5 | 29 | 100 |
| Sucrafer | 50 | 10 | 0 | 0 |

### EXAMPLE 3

Macro- and microscopic evaluation of gastric tolerability in rat.

The test was carried out according to the modified method of Reboani et al.. (Eur. Rev. Med. Pharmacol. Sci. XI, 183, 1989) used to verify FeSO₄ gastric harmlessness.

Male Wistar (Crl: (WI) BR) rats, weighting 250 g and normally fed during the housing period, were used. At the beginning of the experiment, the animals were starved, except water at libitum, in cages with metal grid, without sawdust; after 24 and 48 hours, 5% glucose (0,5 ml/100 g) was administered s.c. The oral treatment with the products under examination started 1 hour after the second glucose administration and was repeated after 5 and 19 hours using a dose of 100 mg as Fe/kg. Control group was treated with 10 ml/kg of distilled water. 5 hours after the last treatment, the animals were sacrificed with ether in excess; each stomach was observed, first externally, then it was removed, opened along the great curve and after washing with distilled H₂O, was mounted on paperboard and, after macroscopic evaluation, was immersed into 10% buffered formaline. Subsequently, the samples were embedded in paraffin, selected, stained with hematossilin-eosine and microscopically examined. The samples were ranked as follows: 0 no lesions; 0.5 probable lesion; 1.0 lesion.

Tables II and III show the results. Sucrafer is practically devoid of gastric lesivity.

**TABLE II**

| (1^{st} experiment) | | | |
|---|---|---|---|
| Treatment | No. of rats | Total score (mean ± s.e.) | |
| | | Macroscopic | Microscopic |
| Controls | 10 | 0.00 ± 0.00 | 0.00 ± 0.00 |
| Ferrous sulfate | 10 | 2.60 ± 0.22** | 4.65 ± 0.51** |
| Sucrafer | 10 | 0.00 ± 0.00 | 0.30 ± 0.13 |

| | | | |
|---|---|---|---|
| ** p < 0.01 Mann Whitney test vs. controls | | | |

**TABLE III**

| (2^{nd} experiment) | | | |
|---|---|---|---|
| Treatment | No. of rats | Total score (mean ± s.e.) | |
| | | Macroscopic | Microscopic |
| Controls | 10 | 0.00 ± 0.00 | 0.00 ± 0.00 |
| Ferrous sulfate | 10 | 1.70 ± 0.20** | 3.55 ± 0.35** |
| Sucrafer | 10 | 0.05 ± 0.05 | 0.00 ± 0.00 |

| | | | |
|---|---|---|---|
| ** p < 0.01 Mann Whitney test vs. controls | | | |

### EXAMPLE 4

### Sideremia in rat

Male Crl: CD (SD)BR rats, weighting 130-150 g, starved for 24 hours, were orally treated with Sucrafer at the dose of 5 mg of Fe/kg. Blood samples were withdrawn in basal conditions and after 1, 2, 4 and 6 hours and heparinized to avoid coagulation. Iron plasma levels were determined by colorimetric method.

The results are shown in Table IV, iron plasma levels are in mg/100 ml (mean ± s.e.).

**TABLE IV**

| | Basal | 1h | 2h | 4h | 6h |
|---|---|---|---|---|---|
| Sucrafer | 46.7 | 201.7 | 228.3 | 100.0 | 52.0 |
| | ±11.6 | ±74.4 | ±58.9 | ±33.5 | ±27.7 |

## Claims

1. The use of saccharose octasulfate ferric complex for the preparation of a medicament for the iron therapy.

## Patentansprüche

1. Verwendung des Saccharoseoctasulfateisenkomplexes für die Zubereitung eines Medikamentes für die Eisentherapie.

## Revendications

1. Utilisation du complexe ferrique d'octasulfate de saccharose pour la préparation d'un médicament pour le traitement de carence de fer.
